# EUROPÄISCHE PATENTSCHRIFT

(11) **EP 0 635 478 B1**
(45) Veröffentlichungstag und Bekanntmachung des Hinweises auf die Patenterteilung: **14.01.1998**
(21) Anmeldenummer: 94110672.6
(22) Anmeldetag: 08.07.1994
(51) Int. Cl.: C07C 69/54, A61K 6/08

(54) **Acrylate und Methacrylate auf Basis von Cyclohexyldiphenolen**
Acrylates and methacrylates based on cyclohexyldiphenols
Acrylates et méthacrylates à base de cyclohexyldiphénols

(30) Priorität: 21.07.1993 DE 4324431
(43) Veröffentlichungstag der Anmeldung: 25.01.1995
(73) Patentinhaber: BAYER AG, 51368 Leverkusen (DE)
(72) Erfinder: Podszun, Wolfgang, Dr., D-51061 Köln (DE); Müller, Michael, Dr., B-2640 Mortsel (BE)

(56) Entgegenhaltungen:
- US-A- 5 109 097
- CHEMICAL ABSTRACTS, vol. 110, no. 18, 1. Mai 1989, Columbus, Ohio, US; abstract no. 155010s, MATSUDA TATSUTO ET AL. 'BISPHENOL DI(METH)ACRYLATES FOR HEAT- AND LIGHT-RESISTANT POLYMERIC MATERIALS.' Seite 8 ;Spalte LINKS ; & JP-A-63 215 653 (NIPPON SHOKUBAI KAGAKU KOGYO CO.,LTD.)
- CHEMICAL ABSTRACTS, Bd. 111, Nr. 2, 10 Juli 1989, Columbus, Ohio, US; Zusammenfassung Nr. 12473E, KAWAGUCHI, M. ET AL. 'Effect of monomer structure on the mechanical properties of light-cured unfilled resins.' Seite 339; & DENT. MATER. J. Bd. 7, Nr. 2, 1988,, Seiten 174 - 181

## Beschreibung

Di(meth)acrylate finden breite Anwendung z.B. als Bestandteil von Kunststofffüllungsmaterialien in der Dentaltechnik.

Ein besonders häufig verwendetes Monomer ist das sogenannte BisGMA der Formel (I)

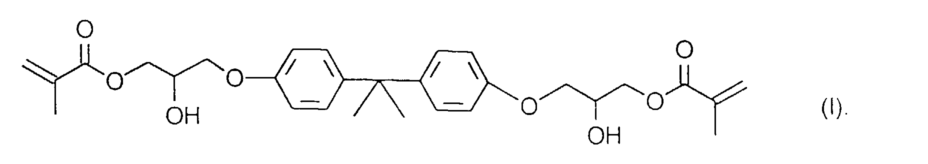

Werkstoffe aus BisGMA weisen ein günstiges Niveau der mechanischen Eigenschaften auf, sie zeigen aber eine deutliche Wasseraufnahme, die für einige Anwendungen, z.B. bei Kunststoffüllungen unerwünscht ist.

Aus der DE-A-4 136 488 sind Monomere der Formel (IIIb) mit n = m = 0 bekannt geworden, die sich als Reaktivverdünner für oxidativ vernetzende Alkydharze in der Lacktechnologie eignen.

Aus Dent. Mater. J. 1988, 7 (2), 174-81 CCA 111: 12473 e, 1988) sind Dimethacrylate beispielsweise des Typs (I) bekannt, worin die Isopropyliden-Brücke durch eine Cyclohexyliden-Brücke ersetzt ist. Es werden die mechanischen Eigenschaften der aus diesen Monomeren hergestellten und durch Licht gehärteten Harze ermittelt und zwar in Relation zur Wasseraufnahme. Fluorgruppen-haltige Dimethacrylatharze sind geeignet als Licht-härtbarer Zahnersatz.

Aus Chem. Abst. Vol. 110, No. 18, 1. Mai 1989, No. 155 010 sind Monomere der Formel (Illb) mit n=m=o und ohne die drei Methylreste an der Cyclohexyliden-Brücke für Polymere für Dentalmaterialien bekannt. Ein Hinweis auf die spezielle Methylsubstitution in (Illb) wird im Chem. Abst. nicht gegeben und auch nicht in der englischen Übersetzung von Sho-63-21 56 53.

Es wurden neue Monomere gefunden, die im gehärteten Zustand eine geringe \Vasseraufnahme und eine erhöhte Glastemperatur aufweisen. Ein weiterer Vorteil der Mehrzahl der erfindungsgemäßen Monomere besteht in ihrer im Vergleich zu BisGMA niedrigeren Viskosität, so daß diese Monomere mit einem reduzierten Anteil an Reaktivverdünner oder ganz ohne Reaktivverdünner eingesetzt werden können.

Die Erfindung betrifft Monomere der Formel (IIIa) 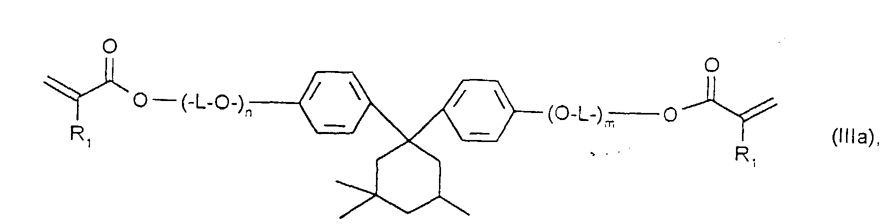 worin
- R₁: für Wasserstoff oder Methyl steht,
- L: für einen C₂- bis C₆-Alkylenrest, der mit Alkyl, Hydroxyl oder Halogen substituiert sein kann, steht,
und
- n und m: unabhängig voneinander eine ganze Zahl von 1 bis 4 bedeuten.

Bevorzugt sind Monomere der Formel (IIIa),
worin
- R₁: für Wasserstoff oder Methyl steht,
- L: für einen C₂- bis C₃-Alkylenrest, der mit Alkyl, Hydroxyl oder Halogen substituiert sein kann, steht,
und
- n und m: unabhängig voneinander 1 oder 2 bedeuten.

Bevorzugte Beispiele für L sind Ethylen, Propylen-1,2 und 2-Hydroxypropylen-1,3. n und m sind besonders bevorzugt 1.

Ein weiterer Gegenstand der vorliegenden Erfindung ist die Verwendung von Monomeren der Formel (IIIb), 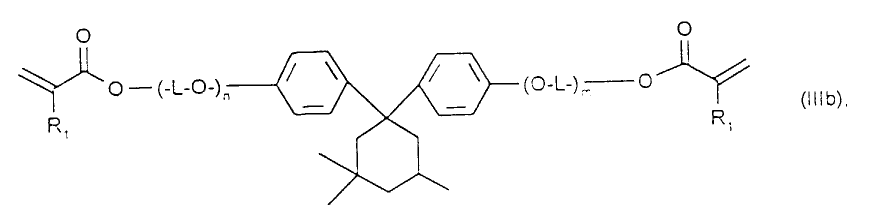 worin
- R₁: für Wasserstoff oder Methyl steht,
- L: für einen C₂- bis C₆-Alkylenrest, der mit Alkyl, Hydroxyl oder Halogen substituiert sein kann, steht,
und
- n und m: unabhängig voneinander eine ganze Zahl von 0 bis 4 bedeuten,
in der Dentaltechnik, insbesondere bei der Herstellung von Kunststoffen zur Verwendung in der Dentaltechnik, z.B. von Kunststoffüllmanen und künstlichen Zähnen, sowie die erhaltenen Dentalobjekte selbst.

Bevorzugt werden Monomere der Formel (IIIb) verwendet, worin
- R₁: für Wasserstoff oder Methyl steht,
- L: für einen C₂- bis C₃-Alkylenrest, der mit Alkyl, Hydroxyl oder Halogen substituiert sein kann, steht,
und
- n und m: unabhängig voneinander eine ganze Zahl von 1 oder 2 bedeuten.

Bevorzugte Beispiele für L sind Ethylen, Propylen-1,2 und 2-Hydroxypropylen-1,3. n und m bedeuten besonders bevorzugt 1.

Die zur bevorzugten Ausführungsform der Erfindung zählenden Monomeren der Formel (IIIa) und die bevorzugte Verwendung von Monomeren (IIIb) ist in der verminderten Kristallisationsneigung der Monomere mit n, m ≥ 1 begründet.

Beispielhaft seien die folgenden erfindungsgemäß verwendbaren Monomere genannt:

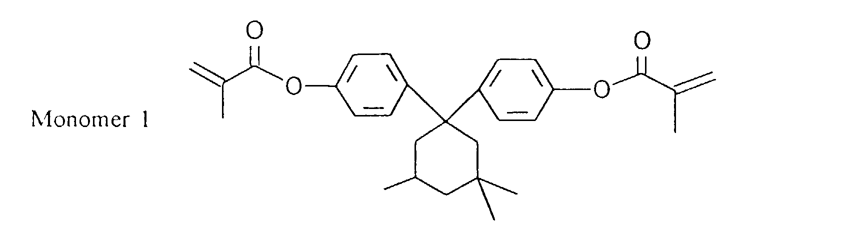

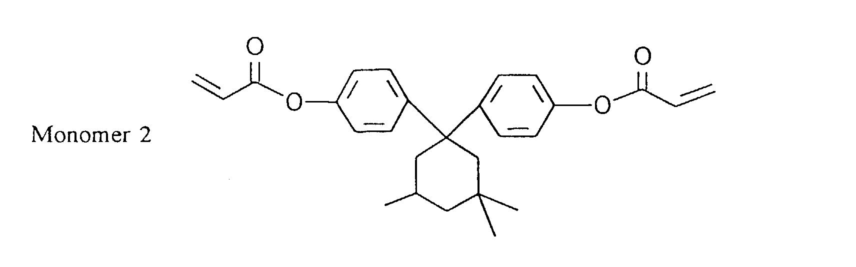

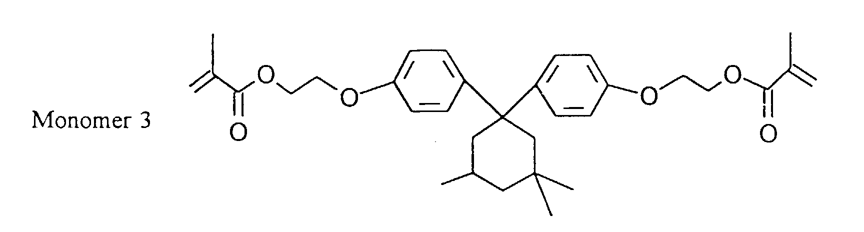

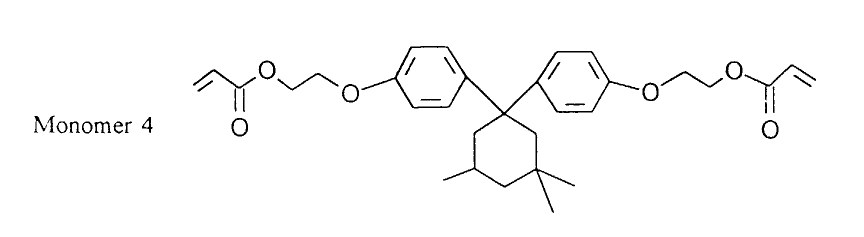

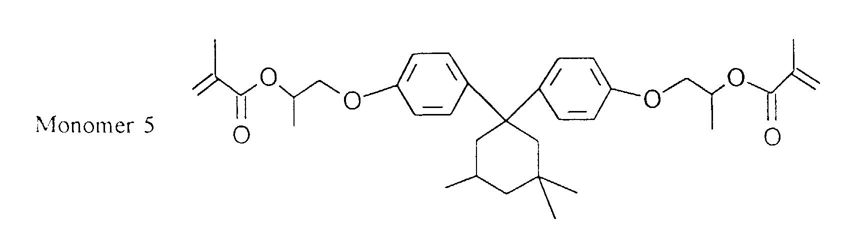

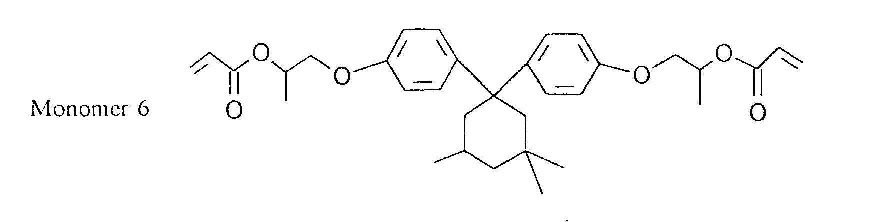

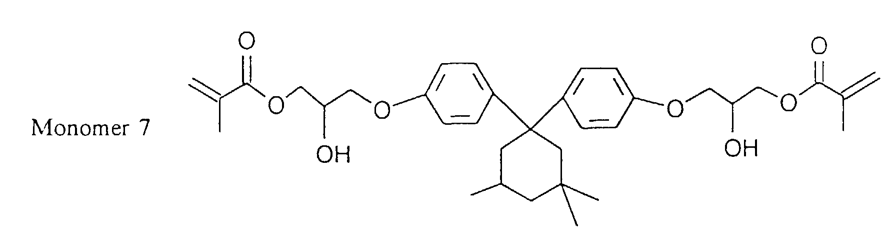

Die Herstellung der erfindungsgemäßen Monomeren erfolgt zweckmäßigerweise aus den zugrundeliegenden Bisphenolen. Monomere vom Typ der Monomere 1 und 2 sind durch Veresterung mit (Meth)acrylsäure oder (Meth)acrylsäureanhydrid bzw. durch Umesterung mit (Meth)acrylsäureestern kurzkettiger Alkohole mit 1 bis 4 C-Atomen im Alkoholteil zugänglich (vgl. auch DE-A-41 36 488). Bevorzugt ist die Veresterung mit (Meth)acrylsäurechlorid. Bei der Synthese der Monomere vom Typ der Monomere 3 bis 6 wird das Bisphenol zunächst unter alkalischer Katalyse mit der gewünschten Menge Ethylenoxid bzw. Propylenoxid umgesetzt und anschließend verestert. Die Synthese von Monomeren vom Typ des Monomers 7 kann entsprechend dem nachfolgenden Schema durchgeführt werden:

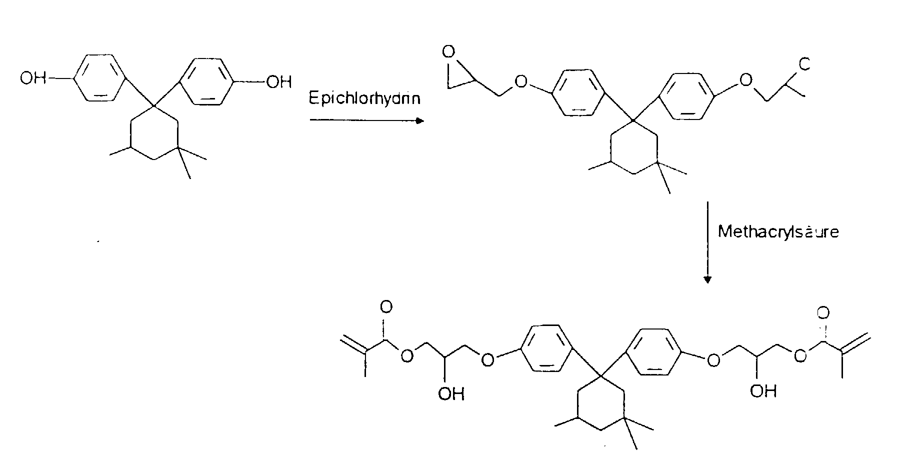

Die (Meth)acrylsäureester der Formel (IIIb) können auch als monomere Komponente für polymerisierbare Zahnfüllmassen eingesetzt werden.

Für diese Anwendung als Monomere für polymerisierbare Zahnfüllmassen im Dentalbereich können die erfindungsgemäßen (Meth)acrylsäureester mit an sich bekannten Monomeren gemischt werden, um beispielsweise die Viskosität dem Verwendungszweck anzupassen. Hierbei werden Viskositäten im Bereich von 60 bis 10.000 mPa.s bevorzugt. Dies ist dadurch erreichbar, daß man den erfindungsgemäßen Monomeren gegebenenfalls ein Comonomer niedriger Viskosität als Reaktivverdünner bzw. Lösungsmittel zumischt. Die erfindungsgemäßen Verbindungen werden in der Mischung mit Comonomeren mit einem Anteil von ca. 30 bis ca. 100 Gew.-%, eingesetzt.

Beispielsweise seien die folgenden Comonomere genannt: Glycerindimethacrylat, Triethylenglykoldimethacrylat, Tetraethylenglykoldimethylacrylat, 1,12-Dodecandioldimethacrylat, 1,6-Hexandioldimethacrylat, Diethylenglykoldimethacrylat, 2,2-Bis-[p-(2'-hydroxy-3'-methacryloyloxypropoxy)-phenyl]-propan, 2,2-Bis-[p-(2'-methacryloyloxyethoxy)-phenyl]-propan, Trimethylol-propan-tri-(meth)acrylat,Bis-(meth)-acryloyloxyethoxymethyl-tricyclo-[5.21,0^{2.6}]-decan (DE-OS 29 31 925 und DE-OS 29 31 926).

Insbesondere bevorzugt werden Comonomere, die bei 13 mbar einen Siedepunkt über 100°C besitzen.

Die erfindungsgemäßen (Methacrylsäureester können gegebenenfalls in Mischung mit den genannten Comonomeren mit an sich bekannten Methoden zu vernetzten Polymerisaten aushärten (Am. Chem. Soc., Symp. Ser. 212, 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen.

Die peroxid- bzw. aminhaltigen Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden Als Initiatoren für die photoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE-PS 3 135 115 beschrieben.

Neben den oben beschriebenen Initiatoren können den erfindungsgemäßen (Meth)-acrylsäure-Derivaten an sich für diesen Einsatzzweck bekannte Lichtschutzmittel und Stabilisatoren zugesetzt werden.

Lichtschutzmittel sind beispielsweise in "Gächter, Müller, Taschenbuch der Kunststoff-Additive, 2. Ausgabe, Carl Hanser Verlag" beschrieben. Beispielsweise seien die folgenden Lichtschutzmittel genannt: Cyasorb UV 9®, Tinuvin P®, Tinuvin 770®, Tinuvin 622®, Tinuvin 765®.

Stabilisatoren sind beispielsweise in "Ullmanns Encyclopädie der technischen Chemie, 4. Auflage, Band 8" beschrieben. Beispielsweise seien die folgenden Stabilisatoren genannt: 2,6-Di-tert. -butylphenol, 2,6-Di-tert.-butyl-4-methylphenol, 2,6-Di-octadecyl-4-methyl-phenol, 1,1'-Methylen-bis(naphthol-2) u.a..

Die Lichtschutzmittel und die Stabilisatoren können jeweils in einer Menge von 0,01 bis 0.5 Gew.-Teilen, bezogen auf 100 Gew.-Teile der Monomermischung, eingesetzt werden.

Die Monomermischungen können ohne Zusatz von Füllstoffen auch als Beschichtungsmittel (Zahnlacke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPa.s besitzen, besonders vorteilhaft.

Vorzugsweise mischt man den erfindungsgemäßen (Meth)acrylsäurederivaten anorganische Füllstoffe zu. Beispielsweise seien Bergkristall, Kristoballit, Quarzglas, hochdisperse Kieselsäure, Aluminiumoxid und Glaskeramiken, beispielsweise Zirkon enthaltende Glaskeramiken (DE-OS 23 740 501) genannt. Die anorganischen Füllstoffe werden zur Verbesserung des Verbunds zur Polymermatrix des Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297-308 (1983)). Bevorzugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen mittleren Teilchendurchmesser und/oder einen unterschiedlichen Silangehalt besitzen.

Der Füllstoffanteil in der Zahnfüllmasse beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen vermischt.

Der Anteil der erfindungsgemäßen (Meth)acrylate in den Zahnfüllmassen beträgt im allgemeinen 10 bis 70 Gew.-% bezogen auf die Füllmasse.

Zahnfüllungsmaterialien, die die erfindungsgemäßen Monomere enthalten, haben einen niedrigen Polymerisationsschrumpf und ergeben Werkstoffe mit guter mechanischer Festigkeit, insbesondere mit einer hohen Härte und hervorragenden Verschleißfestigkeit und zeigen darüber hinaus eine niedrige Wasseraufnahme.

### Beispiele

### Beispiel 1 (Herstellung von Monomer 1)

178,5 g Bis(4-hydroxyphenyl)3,3,5-trimethylcyclohexan, 122 g Triethylamin und 0,30 g 2,6-Di-tert.-Butyl-4-methyl-phenol werden in 1000 ml Methyl-tert.-butylether gelöst. Zu dieser Lösung werden bei -5°C innerhalb von 30 min. 120,2 g Methacrylsäurechlorid zugetropft. Der gebildete Feststoff wird abfiltriert, die etherische Lösung wird 3 mal mit 1000 ml entionisiertem Wasser gewaschen, über Natriumsulfat getrocknet und im Vakuum bei 35°C eingedampft. Man erhält 243 g eines farblosen, kristallinen Feststoffes (Schmelzpunkt: 119°C).

### Beispiel 2 (Herstellung von Monomer 3)

### 1. Stufe, Ethoxylierung

1162,5 g Bis(4-hydroxyphenyl)3,3,5-trimethylcyclohexan und 16,8 g Kaliumhydroxid werden in 1575 g entionisiertem Wasser unter 10 bar N₂-Druck in einem VA-Autoklaven vorgelegt und bei 60°C innerhalb von 10 h mit 924 g Ethylenoxid versetzt. Danach wird 2 h auf 100°C erwärmt. Nach dem Abkühlen werden dem Reaktionsgemisch 2 l Chloroform hinzugefügt. Es entsteht dabei ein zweiphasiges Gemisch. Die organische Phase wird abgetrennt und sieben mal mit 1000 ml entionisiertem Wasser neutral gewaschen, über Natriumsulfat getrocknet und im Vakuum bei 100°C eingedampft. Man erhält 1372 g eines farblosen, glasartig spröden Feststoffes.

### 2. Stufe, Veresterung

200,6 g des Ethoxylierungsproduktes, 111,2 g Triethylamin und 0.30 g 2,6-Di-tert.-butyl-4-methylphenol werden in 1000 ml Dichlormethan gelöst. Zu dieser Lösung werden bei -20 bis -10°C innerhalb von 30 min. 104,5 g Methacrylsäurechlorid zugetropft. Nach beendeter Zugabe wird 1 h bei Raumtemperatur gerührt. Der gebildete Feststoff wird abfiltriert, die etherische Lösung wird 3 mal mit 1000 ml entionisiertem Wasser gewaschen, über Natriumsulfat getrocknet und im Vakuum bei 35°C eingedampft. Man erhält 241 g eines farblosen Feststoffs (Schmelzpunkt: 91°C).

### Beispiel 3 (Herstellung von Monomer 4)

Beispiel 2 wurde wiederholt, wobei anstelle von 104,5 g Methacrylsäurechlorid 90,5 g Acrylsäurechlorid eingesetzt wurden. Man erhält 219 g einer farblosen, viskosen Flüssigkeit.

### Beispiel 4 (Herstellung von Monomer 5)

### 1. Stufe, Propoxylierung

1162,5 g Bis(4-hydroxyphenyl)3,3,5-tfimethylcyclohexan und 16,8 g Kaliumhydroxid werden in 1575 g entionisiertem Wasser in einem VA-Autoklaven vorgelegt mit Stickstoff gespült und bei 60°C innerhalb von 3,5 h mit 609 g Propylenoxid versetzt. Danach wird 2 h auf 100°C erwärmt. Nach dem Abkühlen werden dem Reaktionsgemisch 2 l Chloroform hinzugefügt. Es entsteht dabei ein zweiphasiges Gemisch. Die organische Phase wird abgetrennt und sieben mal mit 1000 ml entionisiertem Wasser neutral gewaschen, über Natriumsulfat getrocknet und im Vakuum bei 50 bis 120°C eingedampft. Man erhält 1239 g eines farblosen, glasartig spröden Feststoffes.

### 2. Stufe, Veresterung

206 g des Propoxylierungsproduktes, 111,2g Triethylamin und 0,30 g 2,6-Di-tert-butyl-4-methylphenol werden in 1000 ml Dichlormethan gelöst. Zu dieser Lösung werden bei -20 bis -10°C innerhalb von 30 min. 104,5 g (Meth)acrylsäurechlorid zugetropft. Nach beendeter Zugabe wird 1 h bei Raumtemperatur gerührt. Der gebildete Feststoff wird abfiltriert, die etherische Lösung wird 3 mal mit je 500 ml entionisiertem Wasser gewaschen, über Natriumsulfat getrocknet und im Vakuum bei 35°C eingedampft. Man erhält 245 g einer leicht gefärbten, klaren, viskosen Flüssigkeit.

## Patentansprüche

1. Monomere der Formel (IIIa) 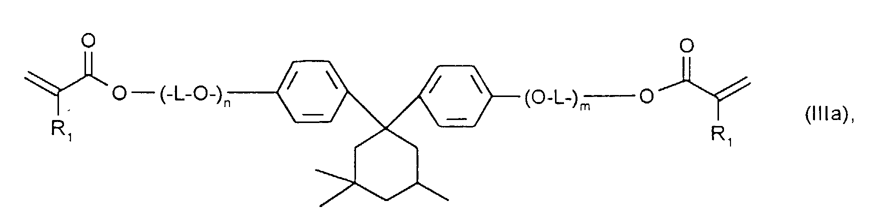 worin
R₁ für Wasserstoff oder Methyl steht,
L für einen C₂- bis C₆-Alkylenrest, der mit Alkyl, Hydroxyl oder Halogen substituiert sein kann, steht,
und
n und m unabhängig voneinander eine ganze Zahl von 1 bis 4 bedeuten.

2. Monomere gemäß Anspruch 1, worin
R₁ für Wasserstoff oder Methyl steht,
L für einen C₂- bis C₃-Alkylenrest, der mit Alkyl, Hydroxyl oder Halogen substituiert sein kann, steht,
und
n und m unabhängig voneinander 1 oder 2 bedeuten.

3. Verwendung der Monomeren der Ansprüche 1 und in der Dentaltechnik.

4. Verwendung der Monomeren der Formel (IIIb) 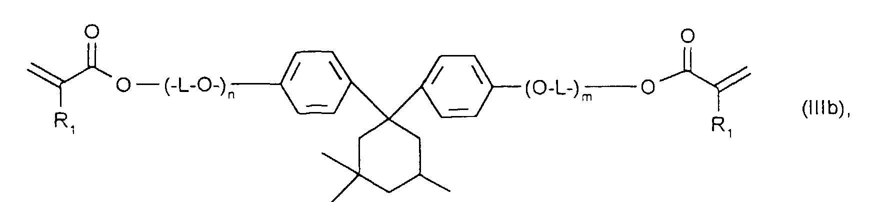 worin
R₁ für Wasserstoff oder Methyl steht,
L für einen C₂- bis C₆-Alkylenrest, der mit Alkyl, Hydroxyl oder Halogen substituiert sein kann, steht,
und
n und m unabhängig voneinander eine ganze Zahl von 0 bis 4 bedeuten
in der Dentaltechnik.

5. Verwendung gemäß Anspruch 4, worin
R₁ für Wasserstoff oder Methyl steht,
L für einen C₂- bis C₃-Alkylenrest, der mit Alkyl, Hydroxyl oder Halogen substituiert sein kann, steht,
und
n und m unabhängig voneinander eine ganze Zahl von 1 oder 2 bedeuten.

6. Verwendung gemäß Ansprüchen 4 und 5 bei der Herstellung von Kunststoffüllmassen in der Dentaltechnik.

7. Dentalobjekte hergestellt aus Monomeren gemäß Ansprüchen 1 und 2.

8. Zahntechnische Kunststoffüllmassen enthaltend Monomere gemäß Ansprüchen 1 und 2.

9. Künstliche Zähne hergestellt unter Verwendung von Monomeren gemäß Ansprüchen 1 und 2.

## Claims

1. Monomers of the formula (IIIa) 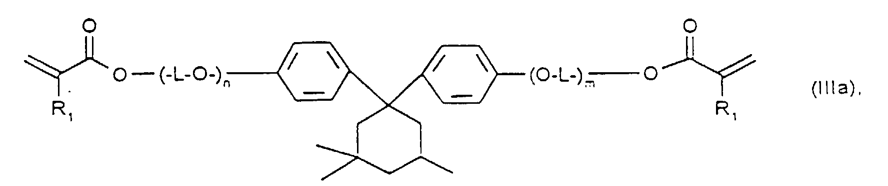 in which
R₁ denotes hydrogen or methyl,
L denotes a C₂ to C₆ alkylene residue, which may be substituted with alkyl, hydroxyl or halogen,
and
n and m mutually independently mean an integer from 1 to 4.

2. Monomers according to claim 1, in which
R₁ denotes hydrogen or methyl,
L denotes a C₂ to C₃ alkylene residue, which may be substituted with alkyl, hydroxyl or halogen,
and
n and m mutually independently mean 1 or 2.

3. Use of the monomers of claims 1 and 2 in dentistry.

4. Use of the monomers of the formula (IIIb) 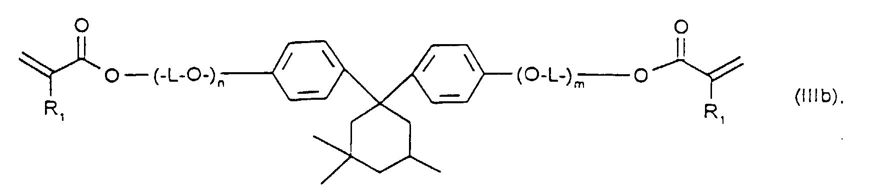 in which
R₁ denotes hydrogen or methyl,
L denotes a C₂ to C₆ alkylene residue, which may be substituted with alkyl, hydroxyl or halogen,
and
n and m mutually independently mean an integer from 1 to 4
in dentistry.

5. Use according to claim 4, in which
R₁ denotes hydrogen or methyl,
L denotes a C₂ to C₃ alkylene residue, which may be substituted with alkyl, hydroxyl or halogen,
and
n and m mutually independently mean an integer of 1 or 2.

6. Use according to claims 4 and 5 in the production of plastic filling compositions in dentistry.

7. Dental objects produced from monomers according to claims 1 and 2.

8. Dental plastic filling compositions containing monomers according to claims 1 and 2.

9. Artificial teeth produced using monomers according to claims 1 and 2.

## Revendications

1. Monomères de formule (IIIa) 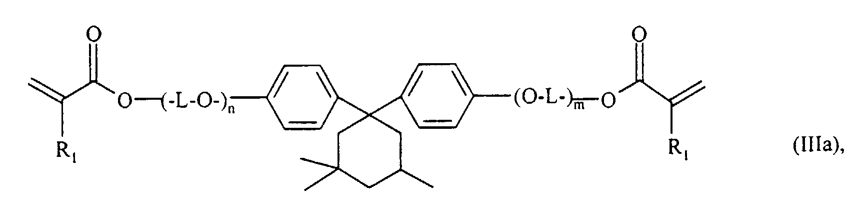 dans laquelle
R₁ représente l'hydrogène ou un groupe méthyle,
L représente un groupe alkylène en C₂-C₆ qui peut être substitué par des groupes alkyles, des groupes hydroxy ou des halogènes, et
n et m représentent chacun, indépendamment l'un de l'autre, un nombre entier allant de 1 à 4.

2. Monomères selon la revendication 1, pour lesquels
R₁ représente l'hydrogène ou un groupe méthyle,
L représente un groupe alkylène en C₂-C₃ qui peut être substitué par des groupes alkyles, des groupes hydroxy ou des halogènes, et
n et m sont égaux chacun, indépendamment l'un de l'autre, à 1 ou 2.

3. Utilisation des monomères des revendications 1 et 2 dans les techniques dentaires.

4. Utilisation des monomères de formule (IIIb) 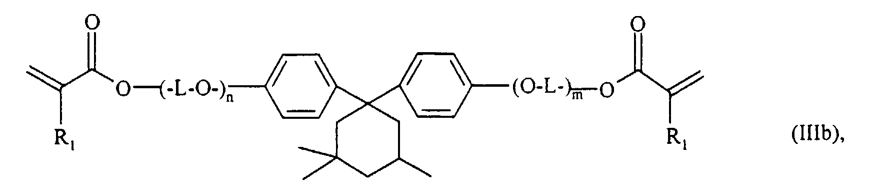 dans laquelle
R₁ représente l'hydrogène ou un groupe méthyle,
L représente un groupe alkylène en C₂-C₆ qui peut être substitué par des groupes alkyles, des groupes hydroxy ou des halogènes, et
n et m représentent chacun, indépendamment l'un de l'autre, un nombre entier allant de 0 à 4,
dans les techniques dentaires.

5. Utilisation selon la revendication 4, pour laquelle
R₁ représente l'hydrogène ou un groupe méthyle,
L représente un groupe alkylène en C₂-C₃ qui peut être substitué par des groupes alkyles, des groupes hydroxy ou des halogènes, et
n et m sont égaux chacun, indépendamment l'un de l'autre, à 1 ou 2.

6. Utilisation selon les revendications 4 et 5 pour la préparation de masses d'obturation en résines synthétiques dans les techniques dentaires.

7. Objets dentaires préparés à partir de monomères selon les revendications 1 et 2.

8. Masses d'obturation en résines synthétiques pour applications dentaires, contenant des monomères selon les revendications 1 et 2.

9. Dents artificielles fabriquées avec utilisation de monomères selon les revendications 1 et 2.
